(19) **Europäisches Patentamt European Patent Office Office européen des brevets**

(11) **EP 2 182 839 B1**

(12) **EUROPEAN PATENT SPECIFICATION**

(45) Date of publication and mention of the grant of the patent:
**26.10.2011 Bulletin 2011/43**

(21) Application number: **07793862.9**

(22) Date of filing: **20.07.2007**

(51) Int Cl.:
*A61B 5/00* (2006.01)     *A61B 5/022* (2006.01)
*A61B 5/029* (2006.01)

(86) International application number:
**PCT/NL2007/050361**

(87) International publication number:
**WO 2009/014419 (29.01.2009 Gazette 2009/05)**

(54) **A CUFF FOR DETERMINING A PHYSIOLOGICAL PARAMETER**

MANSCHETTE ZUR BESTIMMUNG EINES PHYSIOLOGISCHEN PARAMETERS

BRASSARD PERMETTANT DE DÉTERMINER UN PARAMÈTRE PHYSIOLOGIQUE

(84) Designated Contracting States:
**AT BE BG CH CY CZ DE DK EE ES FI FR GB GR HU IE IS IT LI LT LU LV MC MT NL PL PT RO SE SI SK TR**

(43) Date of publication of application:
**12.05.2010 Bulletin 2010/19**

(73) Proprietor: **Bmeye B.V.**
**1105 AZ Amsterdam (NL)**

(72) Inventors:
• **SCHRAA, Olaf**
  **NL-1011 GD Amsterdam (NL)**
• **SCHRAA, Bob**
  **NL-1191 NG Amsterdam (NL)**
• **SETTELS, Jos, J., G., M.**
  **NL-1426 AR De Hoef (NL)**

(74) Representative: **Hatzmann, Martin**
**Vereenigde**
**Johan de Wittlaan 7**
**2517 JR Den Haag (NL)**

(56) References cited:
**WO-A-96/39926     US-A- 4 726 382**

**Description**

FIELD OF THE INVENTION

The invention relates to a cuff for determining a physiological parameter.

BACKGROUND OF THE INVENTION

An embodiment of a cuff as is set forth in the opening paragraph is known from US 4, 726, 382. The known cuff comprises an inflatable bladder formed from a thin flexible, translucent material. The inflatable bladder is connected to a tube enabling a suitable inflation and deflation of the inflatable bladder. In the known cuff the inflatable bladder is the innermost component of the cuff. The inflatable bladder is manufactured from two strips of film being heat sealed together about their periphery thereby forming a cavity. The known cuff is arranged to be fit about a person's finger. For this purpose the inflatable bladder is provided with a back-layer facing outwards from a tissue and a top-layer facing the tissue and being conceived to be brought into contact with the tissue. In order to implement a measurement of a physiological parameter, the known cuff comprises a photoplethysmograph.

In the inflatable bladder of the known cuff openings are provided so that a suitable light source and a light detector can be mounted therein. The inflatable bladder is mounted on a flexible printed circuit. The flexible circuit is used to feed components of the cuff and to supply measurement signals from the cuff to a suitable data processing unit.

It is a disadvantage of the known cuff in that both the back-layer and the top-layer of the inflatable bladder deform to some extent due to applied cuff pressure. This may result in inaccurate reading of physiological parameters such as blood pressure of the cuff due to a changing angle of reflection for a reflective set-up, or a displacement between, for example, the emitter and detector for a transmissive set-up.

SUMMARY OF THE INVENTION

An object of the invention may be to provide a cuff with improved operational characteristics. A further object may be to provide a cuff which can be manufactured in a simplified way. A still further object may be to provide an improvement of or an alternative for a cuff known from the prior art.

According to the invention a cuff according to claim 1 is provided.

Prefered embodiments are defined in the dependent claims.

BRIEF DESCRIPTION OF THE DRAWINGS

Figure 1 presents a schematic elevated view of a cuff according to the invention.
Figure 2 presents a schematic view of the cuff of Figure 1 in a flat condition.
Figure 3 presents a schematic view of the cuff of Figure 1 in use.
Figure 4 presents a schematic view of a measurement system according to the invention.

DETAILED DESCRIPTION

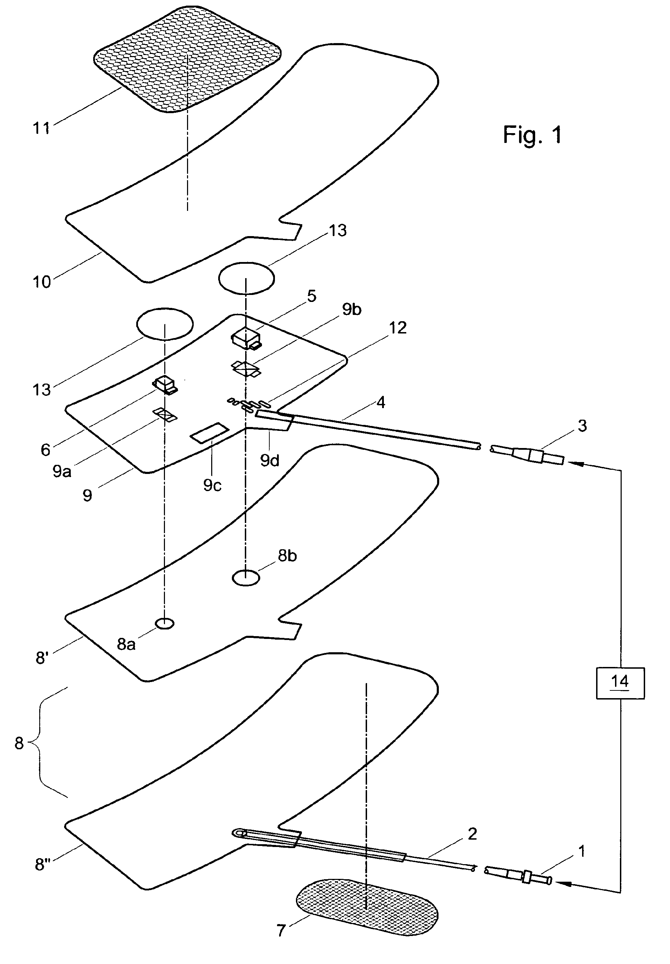

It will be appreciated that the drawings, setting out some aspects of the cuff according to the invention, are not limitative. Figure 1 presents a schematic elevated view of a cuff according to the invention. The cuff comprises an inflatable bladder 8 provided with an air supply channel 2 for inflating the bladder and for evacuating it. For this purpose the air supply channel 2 may comprise a suitable fitting 1 for connecting to a pump, notably a gas pump. The inflatable bladder 8 comprises a top-layer 8" conceived to be brought into contact with a portion of a person, notably with a finger of the person. The inflatable bladder further comprises a back-layer 8' attached to a flexible printed circuit 9. The top-layer is at least more elastic than the back-layer. Elastic should be understood as at least but not limited to any material having a tensile stress at 50% strain of less than 5 MPa. Preferably, the back-layer is substantially or entirely not-elastic. The term non-elastic material refers to at least any material having a yield strength of more than 50 MPa. Presented values hold for a measurement technique of ASTM, DIN or NEN. It is noted that the back-layer is preferably directly attached to the flexible printed circuit 9 without using any additional adhesive inter-layers for reducing a number of components of the cuff. The top-layer 8" may comprise a substantially flexible and elastic material, like polyurethane. The back-layer 8' may comprise a substantially flexible non-elastic material, like polyvinyl chloride. The back-layer 8' may be attached to the top-layer 8" by any suitable technique, preferably a sealing method is used. The back-layer, the flexible printed circuit 9 or the label may be non-elastic. Or two or all of these, whereas the non-elasticity may be provided for or enhanced by other means, such as non-elastic wires incorporated in a longitudinal direction L of the cuff.

The inflatable bladder 8 may further comprise cut-away areas 8a and 8b, wherein a light source 6, such as a light emitting device (LED) and a light detector 5 are positioned, respectively. The back-layer 8' of the inflatable bladder 8 can be attached to the flexible printed circuit 9, which comprises corresponding cut-away areas 9a, 9b for accommodating the light source 6 and the light detector 5. The flexible printed circuit 9 may further comprise suitable blockers 13 for shielding the light source 6 and the detector 5 from interference with other light sources or detectors. Preferably, the blockers 13 comprise opaque flexible material. A signal from the light detector 5 is picked up by one or more suitable electronic components (not shown) of the flexible printed circuit 9. The flexible printed circuit 9 is electrically connectable to a cable 4 provided with a suitable electric connector 3. It is possible that the cable 4 and the air supply 2 are housed in a joint housing having a single outside connector 14. The flexible printed circuit may comprise an identifying unit 12. Preferably, the flexible printed circuit 9 further comprises a module 9c for processing the signal from the detector 5. Suitable signal processing steps performed by the computers and/or a processing unit to be connected to the cable 4 may comprise a filtering, amplification, or the like.

A cuff 20 according to the invention may further comprise a sticker 10, which may comprise or form a suitable label. Fastening means, such as loop 11 and hook 7 material, for example Velcro® may be arranged on the cuff 20 to fasten the cuff about a portion of a recipient, for example around a finger of a person. Preferably, the top-layer 8" is manufactured from a biocompatible material and extends substantially over the same length as the back-layer 8' or the sticker 10. Due to the fact that only the biocompatible top-layer is in contact with the tissue, the label or the back-layer does not have to be manufactured from a biocompatible material reducing the production costs of the cuff.

Preferably, a surface of the flexible printed circuit conceived to face the tissue in use comprises a coating, preferably a coating which is electrically conductive and optically opaque and/or reflective. This can have an effect that the light that may be reflected from the tissue and impinges on the coating, will be reflected back towards the tissue, improving signal-to-noise ratio of the measurement signal. In addition, such coating may enable proper electrical shielding of the components of the flexible printed circuit. The opacity of the flexible circuit material may advantageously prevent the detector of the photoplethysmograph from interference of ambient light with the photoplethysmograph, which also contributes to an improvement of the signal-to-noise ratio of the cuff according to the invention. Preferably, metal traces, notably copper traces, are used in the flexible printed circuit to connect the electrical cable 4 to the components of the flexible printed circuit, which makes wiring redundant, further decreasing manufacturing costs of the cuff according to the invention.

In a further embodiment of the cuff, the flexible printed circuit is shaped with a tail-end 9d, such as a projection, for relieving fastening strain applied to the cabling 4 and the air duct 2 in use. This improves durability of the cuff, particularly when it is conceived to be repetitively fastened and removed.

The cuff 20 is conceived to be arranged on a suitable portion of a body, for example around a finger, on an ear or the temples of the head, in a nostril, or in a body cavity. The cuff 20 comprises a photoplethysmograph arranged with an emitter 6, for example a LED or an infra-red source, and a light detector 5, for example a photodiode. This emitter-receiver pair is used to determine a blood flow in said portion of the body. In case a transmissive set-up is used, a light emitting diode may be used to transmit light through the skin. The detector 5 picks up the transmitted signal, which can then be analyzed using suitable signal processing techniques. A signal from the photoplethysmograph comprises a pulse wave corresponding to changes in blood volume in the arteries or capillaries receiving the light from the emitter 6. Changes in blood volume produce changes in optical absorption of the emitted light. The light transmitted through the tissue can be highly scattered or absorbed depending on the tissue. The detector, which is positioned on the surface of the skin, can detect the transmission of waves from various depths and from highly absorbing or weakly absorbing tissues. Regardless of the absorbency of the tissues and skin, it is generally assumed that the amount of light absorbed and/or reflected by these tissues will remain constant. With this assumption in mind, it can then be assumed that the only change in the absorption or reflection of the transmitted light will be from the increase or decrease of the blood volume in the arteries and capillaries. The measured volume change is actually an average of all of the arteries and capillaries in the space being irradiated. For a transmissive set-up infrared radiation may be chosen for the emitter 6 because infrared is well absorbed by blood and very weakly absorbed by other tissues and fluids in the body. This means that the blood volume changes can be very easily observed. Preferably, for detecting the signal an infrared receiver is chosen, sensitivity of which matches at least partially the spectrum of the infrared emitter. It may also be beneficial to use an infrared emitter because changes in blood oxygen content are very prominent in the visible light region. Due to the fact that the inflatable bladder comprises a top-layer which is more elastic than the back-layer, measurements of a physiological parameter such as blood pressure are obtained with improved accuracy, because less or no distortion of a light path between the light source and the light detector is caused by a deformation of the back-layer.

The placement of the emitter and receiver on the body is also an important aspect of the photoplethysmograph. It may be chosen to position the photoplethysmograph at an earlobe or at the fingers, because of the consistency of the tissues. These areas on the body can also be held relatively still to reduce motion artifact which would otherwise distort the output signal. Still another applicable area may be the nasal septum. However, different positions are possible.

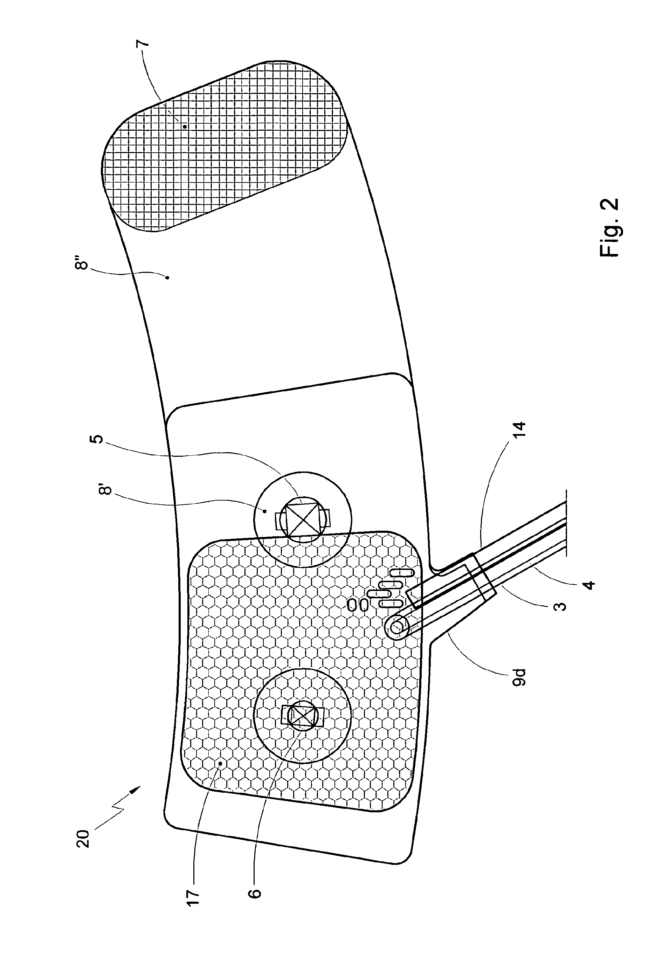

Figure 2 presents a schematic view of the cuff of Figure 1 in a flat condition. The cuff 20 is shown in a top view, revealing a substantially transparent top-layer 8" where through a back-layer 8' is seen provided with cut-way areas for accommodating respective parts of the photoplethysmograph, notably the light source 6 and the light detector 5. The cuff 20 can be inflated using air supply duct 2. Measurement data from the photoplethysmograph can be collected using a suitable electric cable 4. The electric cable 4 and the air supply channel 2 may be arranged in a suitable tube 14 which is attached to the cuff 20 by means of a tail portion 9d. The cuff 20 may be conceived to be wrapped around a body portion, for example a finger, in this case the cuff 20 may comprise fastening means, for example hooks 7 cooperating with loops 17.

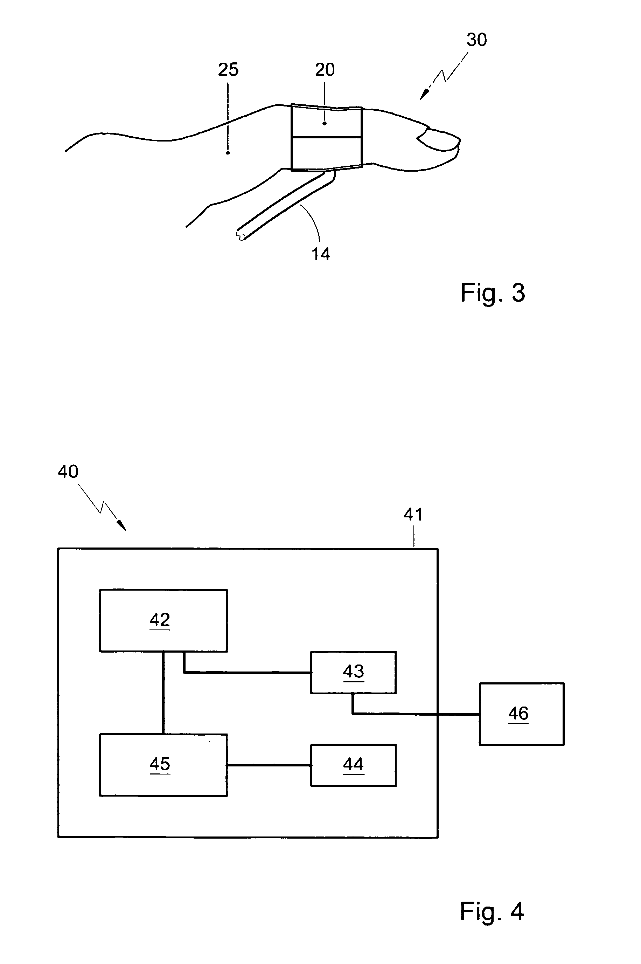

Figure 3 presents a schematic view 30 of the cuff of Figure 1 in use, wherein the cuff 20 is wound about a finger 25 of a person under investigation. It is noted that especially when the top-layer of the cuff 20 comprises a biocompatible material, the cuff 20 may be used for substantially prolonged period of time on the person, for example for purposes of durable monitoring of a vital sign, notably of pressure and/or cardiac output.

Figure 4 presents a schematic view of a measurement system according to the invention. The system 40 comprises a processor 41 and a measurement unit 46, which may preferably comprise the cuff for carrying measurements of the arterial pressure waveform, as is set forth with reference to Figure 1. Data collected by the measurement unit 46 is provided to the input 43 of the processor 41. It is noted that using the measurement unit 46, for example the cuff as is discussed with reference to Figure 1, a plurality of useful signals may be acquired. For example, plethysmogram and blood pressure data may be used for determining in non-invasive way additional information, for example using suitable models applicable to said data. For example, cardiac output can be determined non-invasively using arterial data provided by the measurement unit 46. The processor 41 further comprises storage 42 for storing a suitable model, for example a non-linear model approximating a relation between an aortic cross-section and applied pressure. Preferably, for the non-linear model an arctangent model is selected.

The aortic mechanical properties approximate a response of the internal cross-sectional area of the aorta to an increase in pressure by an arctangent:

$$A(p) = Am \ (0.5 + (1/\pi) \ \arctan((p\text{-}p0)/p1))$$

in which A the cross-sectional area in cm$^2$;
Am the maximal area at very high pressure;
p0 indicating the inflexion point of a pressure curve;
p1 indicating a halfwidth of a pressure pulse.

The processor 41 further comprises a computing unit 45 arranged for computing a compliance and/or an impedance of an aortic portion from the acquired arterial pressure data. For obtaining the compliance or the impedance the non-linear model, notably the arctangent model, is differentiated. The thus obtained value of the compliance or impedance may be incorporated into per se known pulse contour method, like for example the Waterhammer model or Windkessel model, for determining the beat-to-beat stroke volume and/or cardiac output based on the measured arterial pressure data, notably the waveform.

Windkessel model is a linear model pulse contour model that describes a relation between a stroke volume Vs, aortic compliance and characteristics of the waveform of arterial pressure.

$$Vs = C \ (p2 \text{ - } p1) \ (1 + As/Ad)$$

with Vs - a stroke volume, C - an aortic compliance, defined as dV/dP, p2 - a pressure at a dicrotic notch, p1 the diastolic pressure, As the integrated area under the systolic portion of the blood pressure curve, and Ad similarly the diastolic area. The dicrotic notch is a pulse that precedes a dicrotic wave, it being a pulse sequence comprising a double-beat sequence wherein a second beat is weaker than a first beat.

Waterhammer model is another linear pulse contour model that describes a relation between impedance Zc, density of blood, aortic cross-sectional area and aortic compliance:

$$Zc = \surd \ (r \ / \ (A \ C'))$$

with r the density of blood, A the aortic cross-sectional area and C' the compliance per unit length.

It is noted that the cardiac output equals the stroke volume multiplied by a heart beat frequency. In this way a system is provided for determining beat-to-beat stroke volume and/or cardiac output based on the measurement of an arterial pressure waveform with increased accuracy compared to prior art.

It is found to be advantageous to provide a-priori tabulated relationships between theses parameters and patient characteristics, like age and gender. The computing unit 45 may be arranged to use these tabulations to calculate the compliance data from the arctangent model, yielding:

$$C(p) = Cm / ( 1 + ((p\text{-}p0)/p1)^2 )$$

with

$Cm = Am/(\pi\ p1)$,

wherein

$C(p)$ is a pressure-dependent compliance;

$Cm$ - is a maximum compliance or the aortic portion.

The computing means 45 is then arranged to incorporate the calculated value of the compliance in a linear model to calculate the beat-to beat stroke volume and/or cardiac output.

While specific embodiments have been described above, it will be appreciated that the invention may be practiced otherwise than as described. The descriptions above are intended to be illustrative, not limiting. Thus, it will be apparent to one skilled in the art that modifications may be made to the invention as described in the foregoing without departing from the scope of the claims set out below.

**Claims**

1. A cuff (20) for determining a physiological parameter, comprising:

    - a photoplethysmograph arranged with
    - an emitter (6) for emitting a radiation in a direction of a tissue to be investigated;
    - a detector (5) for detecting the radiation from the tissue;
    and
    - an inflatable bladder (8) for transferring pressure to the tissue, said inflatable bladder comprising a back-layer (8') and a top-layer (8"),
    wherein the top-layer (8") is conceived to be brought into contact with the tissue, comprises a flexible substantially elastic material and is substantially more flexible than the back-layer (8') **characterized in that**, back-layer (8') and the top-layer (8") have substantially the same thickness, and the back-layer (8') comprises a substantially non-elastic material

2. A cuff (20) according to claim 1, wherein the top-layer (8") is arranged to provide an electrical insulation between the tissue and electrical components of the cuff.

3. A cuff (20) according to any preceding claim, wherein the top-layer (8") is arranged to protect electrical components of the cuff from contamination, like dirt and/or sweat.

4. A cuff (20) according to any one of the preceding claims, wherein the top-layer (8") is at least partially manufactured from a biocompatible material.

5. A cuff (20) according to claim 4, wherein the top-layer (8") extends further than an area of the inflatable bladder (8) conceived to come into contact with the tissue in use.

6. A cuff (20) according to any one of the preceding claims, wherein the back-layer (8') is provided with cut-away areas for transmitting the radiation from the emitter (6) to a tissue and/or for transmitting a further radiation from the tissue to the detector (5).

7. A cuff (20) according to any one of the preceding claims, wherein the emitter (6) is arranged for emitting a focused beam of radiation.

**8.** A cuff (20) according to any one of the preceding claims, wherein the emitter (6) comprises a built-in opaque shield (13).

**9.** A cuff (20) according to any one of the preceding claims, wherein the emitter (6) is arranged to emit radiation with a wavelength in a range of 660 - 1000 nm.

**10.** A cuff (20) according to any one of the preceding claims, wherein the detector (5) comprises a photodiode arranged to be sensitive for a radiation with a wavelength matching at least part of a spectrum of the emitter (6).

**11.** A cuff (20) according to any one of the preceding claims, further comprising:

- an air tube (2) communicating with the inflatable bladder (8);
- means for preventing blockage of an airflow in the air tube.

**12.** A cuff (20) according to claim 11, wherein the air tube (2) is cut at an angle less than 90 degrees and is mounted in the inflatable bladder (8) with the longest projection towards the top-layer (8").

**13.** A measurement system comprising a cuff (20) according to any one of the preceding claims and a data processing unit (40).

**14.** A measurement system according to claim 13, wherein the data processing unit (40) is arranged to:

- compute a compliance or impedance in dependence of at least one measurement of arterial pressure data and patient data using a non-linear model;
- use said compliance or impedance in a pulse contour method for determining the beat-to-beat stroke volume and/or cardiac output based on the measured arterial pressure data.


**Patentansprüche**

**1.** Manschette (20) zum Bestimmen eines physiologischen Parameters, umfassen:

- einen Photoplethysmographen, der angeordnet ist mit
- einem Emitter (6) zum Emittieren von Strahlen in der Richtung eines zu untersuchenden Gewebes;
- einem Detektor (5) zum Detektieren der Strahlung von dem Gewebe; und
- einer aufblasbaren Blase (8) zum Übertragen von Druck auf das Gewebe, wobei die aufblasbare Blase einer Unterschicht (8') und eine Oberschicht (8") umfasst, wobei die Oberschicht (8") dazu ausgelegt ist, mit dem Gewebe in Kontakt gebracht zu werden und ein flexibler, weitgehend elastisches Material umfasst und im Wesentlichen flexibler als die Unterschicht (8') ist, **dadurch gekennzeichnet, dass** die Unterschicht (8') und die Oberschicht (8") im Wesentlichen die gleiche Dicke haben und dass die Unterschicht (8') ein weitgehend nichtelastisches Material enthält.

**2.** Manschette (20) nach Anspruch 1, wobei die Oberschicht (8") so angeordnet ist, dass sie eine elektrische Isolierung zwischen dem Gewebe und den elektrischen Bauteilen der Manschette bereitstellt

**3.** Manschette (20) nach einem der vorhergehenden Ansprüche, wobei die Oberschicht (8") so angeordnet ist, dass sie elektrische Bauteile der Manschette vor Verschmutzungen wie Schmutz und/oder Schweiß schützt,

**4.** Manschette (20) nach einem der vorhergehenden Ansprüche, wobei die Oberschicht (8") zumindest teilweise aus einem biokompatiblen Material hergestellt ist.

**5.** Manschette (20) nach Anspruch 4, wobei die Oberschicht (8") sich über einen Bereich der aufblasbaren Blase (8) hinauserstreckt, der dazu ausgelegt ist, während der Benutzung in Kontakt mit dem Gewebe zu treten,

**6.** Manschette (20) nach einem der vorhergehenden Ansprüche, wobei die Unterschicht (8') mit ausgeschnittenen Bereichen zur Übertragung von Strahlung von dem Emitter (6) auf ein Gewebe und/oder zum Übertragen einer weiteren Strahlung von dem Gewebe an den Detektor (5) ausgestattet ist.

7. Manschette (20) nach einem der vorhergehenden Ansprüche, wobei der Emitter (6) dazu ausgelegt ist, einen fokussierten Strahl der Strahlung zu emittieren.

8. Manschette (20) nach einem der vorhergehenden Ansprüche, wobei der Emitter (6) eine eingebaute, lichtundurchlässige Abschirmung (13) umfasst.

9. Manschette (20) nach einem der vorhergehenden. Ansprüche, wobei

10. Manschette (20) nach einem der vorhergehenden Ansprüche, wobei der Detektor (5) eine Photodiode umfasst, die so ausgelegt ist, dass sie empfindlich für Strahlung mit einer Wellenlänge ist, die mit wenigstens einem Teil des Spektrums des Emitters (6) übereinstimmt.

11. Manschette (20) nach einem der vorhergehenden Ansprüche, ferner umfassend:

   - eine Luftröhre (2), die mit der aufblasbaren Blase (8) kommuniziert;
   - Mittel zum Vermeiden einer Blockade des Luftflusses in der Luftröhre.

12. Manschette (20) nach Anspruch 11, wobei die Luftröhre (2) in einem Winkel mit weniger als 90 Grad abgeschnitten und so in der aufblasbaren Blase (8) montiert ist, dass der längste Vorsprung in Richtung der Oberschicht (8") ausgerichtet ist.

13. Messsystem umfassend eine Manschette (20) nach einem der vorhergehenden Ansprüche und einer Datenverarbeitungseinheit (40).

14. Messsystem nach Anspruch 13, wobei die Datenverarbeitungseinheit (40) ausgelegt ist zum:

   - Berechnen einer Nachgiebigkeit oder eines Widerstands in Abhängigkeit von zumindest einer Messung von Arteriendruckdaten und Patientendaten unter der Verwendung eines nicht-linearen Modells;
   - Benutzen der Nachgiebigkeit oder des Widerstands in einem Pulskonturverfahren zur Ermittlung des Schlag-zu-Schlag-Volumens und/oder der Herzleistung auf der Grundlage der gemessenen Arteriendruckdaten.


**Revendications**

1. Brassard (20) permettant de déterminer un paramètre physiologique, comprenant :

   un photopléthysmographe comportant :

   un émetteur (6) pour émettre un rayonnement dans la direction d'un tissu devant être examine ;
   un détecteur (5) pour détecter le rayonnement venant du tissu ;
   et
   une vessie gonflable (8) pour transférer une pression sur le tissu, ladite vessie gonflable comprenant une couche arrière (8') et une couche supérieure (8"),
   dans lequel la couche supérieure (8") est conçue pour être mise en contact avec le tissu, comprend un matériau flexible sensiblement élastique et est sensiblement plus flexible que la couche arrière (8'), **caractérisé en ce que** la couche arrière (8') et la couche supérieure (8") ont sensiblement la même épaisseur, et la couche arrière (8') comprend un matériau sensiblement non élastique.

2. Brassard (20) selon la revendication 1, dans lequel la couche supérieure (8") est prévue pour former une isolation électrique entre le tissu et les composants électriques du brassard.

3. Brassard (20) selon l'une quelconque des précédentes revendications, dans lequel la couche supérieure (8") est prévue pour protéger les composants électriques du brassard contre une contamination telle que des impuretés et/ou de la sueur.

4. Brassard (20) selon l'une quelconque des précédentes revendications, dans lequel la couche supérieure (8") est au moins partiellement faite d'un matériau biocompatible.

**5.** Brassard (20) selon la revendication 4, dans lequel la couche supérieure (8") s'étend au-delà d'une surface de la vessie gonflable (8) conçue pour venir en contact avec le tissu lors de l'utilisation.

**6.** Brassard (20) selon l'une quelconque des précédentes revendications, dans lequel la couche arrière (8') est munie de zones découpées pour transmettre le rayonnement depuis l'émetteur (6) vers un tissu et/ou pour transmettre un autre rayonnement depuis le tissu vers le détecteur (6).

**7.** Brassard (20) selon l'une quelconque des précédentes revendications, dans lequel l'émetteur (6) est prévu pour émettre un faisceau focalisé de rayonnement.

**8.** Brassard (20) selon l'une quelconque des précédentes revendications, dans lequel l'émetteur (6) comprend un écran opaque incorporé (13).

**9.** Brassard (20) selon l'une quelconque des précédentes revendications, dans lequel l'émetteur (6) est prévu pour émettre un rayonnement ayant une longueur d'onde comprise dans une gamme de 660 à 1000 nm.

**10.** Brassard (20) selon l'une quelconque des précédentes revendications, dans lequel le détecteur (5) comprend une photodiode prévue pour être sensible à un rayonnement ayant une longueur d'onde correspondant à au moins une partie d'un spectre de l'émetteur (6).

**11.** Brassard (20) selon l'une quelconque des précédentes revendications, comprenant en outre :

   un tuyau d'air (2) communiquant avec la vessie gonflable (8) ;
   des moyens pour empêcher le blocage d'un écoulement d'air dans le tuyau d'air.

**12.** Brassard (20) selon la revendication 11, dans lequel le tuyau d'air (2) est coupé suivant un angle inférieur à 90 degrés et est monté dans la vessie gonflable (8) avec la partie la plus longue dirigée vers la couche supérieure (8").

**13.** Système de mesure comprenant un brassard (20) selon l'une quelconque des précédentes revendications et une unité de traitement de données (40).

**14.** Système de mesure selon la revendication 13, dans lequel l'unité de traitement de données (40) est prévue pour :

   calculer une compliance ou une impédance en fonction d'au moins une mesure de données de pression artérielle et de données de patient à l'aide d'un modèle non linéaire ;
   utiliser ladite compliance ou impédance dans un procédé de contour de l'onde de pouls pour déterminer le volume systolique battement par battement et/ou le débit cardiaque en se basant sur les données de pression artérielle mesurées.

Fig. 1

11

10

13

13

5

9b

12

6

9a

9

9c

9d

4

3

14

8b

8'

8a

8

8"

2

1

7

Fig. 2

25    20

30

14

Fig. 3

40

41

42

43

46

45    44

Fig. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4726382 A